# NEUE EUROPÄISCHE PATENTSCHRIFT

(11) **EP 2 196 164 B2**
(45) Veröffentlichungstag und Bekanntmachung des Hinweises auf die Entscheidung über den Einspruch: **10.09.2025**
(45) Hinweis auf die Patenterteilung: 19.10.2022
(21) Anmeldenummer: 09013659.9
(22) Anmeldetag: 30.10.2009
(51) Int. Cl.: A61C 8/00, A61C 3/02

(54) **Set von Dentalbohrern**
Set of dental drills
Ensemble de fraises dentaires

(30) Priorität: 15.12.2008 CH 20322008
(43) Veröffentlichungstag der Anmeldung: 16.06.2010
(73) Patentinhaber: Straumann Holding AG, 4002 Basel (CH)
(72) Erfinder: Suter, Edmund, 4435 Niederdorf (CH); Kühne, Steffen, 4313 Möhlin (CH); Streff, Patrick, 79576 Weil am Rhein (DE)
(74) Vertreter: Schaad, Balass, Menzl & Partner AG

(56) Entgegenhaltungen:
- EP-A2- 0 726 065
- DE-U1- 202004 000 723
- US-A1- 2005 170 311
- NOBELDIRECT GROOVY PROCEDURES & PRODUCTS
- ANONYMOUS: "NobelDirect, Clinical Procedure & Product Catalog", NOBEL BIOCARE, 1 February 2004 (2004-02-01), pages 1 - 36, XP093129232

## Beschreibung

Die vorliegende Erfindung betrifft ein Set von Dentalbohrern.

In der Dentalimplantologie ist es bekannt, für die Präparation eines Implantatbettes einen Satz von mehreren verschiedenen Dentalbohrern zu verwenden, welches beispielsweise einen Rosenbohrer, einen Pilotbohrer, einen Profilbohrer, einen Spiralbohrer und einen Gewindebohrer umfasst.

In groben Zügen ist das Vorgehen bei der Präparation eines Implantatbettes wie folgt: Als erstes wird die Knochenoberfläche freigelegt und mit einem Rosenbohrer geglättet. Die Implantationsstelle wird mit dem Rosenbohrer markiert und möglicherweise erweitert, wobei im letzteren Fall mehrere Rosenbohrer mit aufsteigenden Durchmessern eingesetzt werden. Anschliessend wird das Implantatbett mit geeigneten Pilotbohrern zur Bestimmung der Bohrachse präpariert. Mittels Spiralbohrern mit aufsteigenden Durchmessern wird das Bohrloch auf die gewünschte Grösse erweitert. Ein Profilbohrer dient dazu, das Profil des Bohrlochs an die Form des vorgesehenen Implantats anzupassen. In einem abschliessenden Schritt wird dann mit Hilfe eines Gewindebohrers ein Innengewinde im Bohrloch angebracht.

Um eine optimale Implantationsachse zu gewährleisten, wird für die Präparation des Implantatbettes üblicherweise eine Bohrschablone, auch "Template" genannt, verwendet. Solche Bohrschablonen dienen dazu, Dentalbohrer zu führen und ein Abrutschen oder Abknicken während des Bohrprozesses zu verhindern. Eine Bohrschablone ist derart ausgestaltet, dass sie für die Präparationen von einer oder mehreren Implantationsstellen verwendet werden können. Sie weist ein oder mehrere Bohrlöcher auf, in welche die Dentalbohrer vor dem Bohren eingeführt werden. Oft werden standardisierte Metallbohrhülsen in die Bohrlöcher der Bohrschablone eingebracht, beispielsweise indem sie einpolymerisiert oder eingepresst werden, um die Präzision der Bohrung weiter zu erhöhen. Ein solches Verfahren ist zum Beispiel in WO 99/26540 offenbart.

US 2005/170311 A1 offenbart ein Set aus Stufenbohrer und Kalibrierbohrer sowie ein Verfahren und eine Vorrichtung zum Einsetzen von Implantaten unter Verwendung einer Bohrschablone. Die Bohrschablone umfasst mehrere Bohrlöcher, in welche Bohrhülsen eingesetzt werden, die als Bohrerführung dienen. Die Bohrhülsen weisen einen Standard-Innendurchmesser auf und die Stufenbohrer sowie Kalibrierbohrer weisen einen Führungsbereich mit einem entsprechenden Standard-Aussendurchmesser auf.

DE 20 2004 000723 U1 betrifft einen Pilotbohrer, einen Stufenbohrer und ein daraus gebildetes Bohrerset zur Verwendung in der Dentalimplantologie. Der Pilotbohrer hat an seinem apikalen Ende eine Pilotspitze mit Spitzenschneiden, von welcher sich in Richtung des koronalen Endes des Pilotbohrers eine Pilotführung erstreckt. Oberhalb der Pilotführung liegt ein Bohrhals, der einen grösseren Bohrerdurchmesser aufweist als der Bohrerdurchmesser der Pilotführung. Seitlich an der Pilotführung liegt zumindest eine Führungsschneide, die stumpf, also nicht-schneidend gestaltet ist. Im Übergang von der Pilotführung zum Bohrhals befindet sich eine Stufe mit zumindest einer Stufenschneide, die schneidend ausgebildet ist.

EP 0 726 065 A2 offenbart ein Drehbohrinstrument für Zahnbehandlungen, zur Verbindung mit einem Drehwerkzeug im Gebrauch, umfassend einen ein spiralförmiges Messer aufweisenden Schneidabschnitt, einen an den Schneidabschnitt anschliessenden Halsabschnitt und einen an den Halsabschnitt anschliessenden Schaft. Der Durchmesser des Halsteils ist kleiner als der Durchmesser des Schneidabschnitts und als der Durchmesser des Schafts.

Im Laufe des Bohrprozesses müssen die Bohrlöcher oder Bohrhülsen in der Bohrschablone üblicherweise durch entsprechende Reduktionshülsen an den Durchmesser des gerade verwendeten Dentalbohrers angepasst werden. Alternativ können auch passende Bohrlöffel in die Bohrlöcher oder Bohrhülsen eingesetzt werden. Für die vollständige Präparation eines einzelnen Implantatbettes ist also ein ganzes Set von unterschiedlichen Reduktionshülsen oder Bohrlöffeln notwendig. Zudem müssen die Reduktionshülsen oder Bohrlöffel laufend ausgewechselt werden, was zu einem erhöhten Aufwand und Fehlerpotential führt.

Bei einzelnen Dentalbohrern, insbesondere bei Profilbohrern, kann es ausserdem vorkommen, dass diese zu breit sind für eine solche Reduktionshülse oder einen Bohrlöffel. In diesem Fall muss die Bohrung ohne Führung durchgeführt werden und das Risiko für eine Bohrungenauigkeit ist deutlich grösser.

Es ist daher eine Aufgabe der vorliegenden Erfindung, die Führung der Dentalbohrer während des gesamten Bohrerprozesses zu vereinfachen und für besonders breite Dentalbohrer überhaupt zu ermöglichen.

Diese Aufgabe wird gelöst durch das Dentalimplantologieset nach Anspruch 1. Bevorzugte Ausführungsformen sind Gegenstand der abhängigen Ansprüche.

Das Dentalimplantologieset umfasst ein Set von Dentalbohrern, welches zwei oder mehrere Dentalbohrer umfasst. Die Dentalbohrer weisen ein Schneideteil mit einem stirnseitigen Bohrende und einen Schaftteil mit einem Aufnahmeendbereich auf, welcher dazu bestimmt ist, in einer Bohrerhaltevorrichtung aufgenommen zu werden. Die Dentalbohrer sind dadurch gekennzeichnet, dass der Schaftteil einen im Wesentlichen kreiszylinderförmigen Führungsbereich aufweist zur Führung des Dentalbohrers und dass die Führungsbereiche der zwei oder mehr Dentalbohrer den gleichen Durchmesser aufweisen.

Dadurch, dass die Führungsbereiche der Dentalbohrer einen identischen Durchmesser haben, ist es nicht nötig, die Reduktionshülse oder den Bohrlöffel während des Bohrprozesses zu wechseln. Der Durchmesser des Führungsbereichs der einzelnen Dentalbohrer und damit der nötige Innendruchmesser der Bohrerführung ist also unabhängig vom Durchmesser des Schneideteils der Dentalbohrer. Da die Reduktionshülsen während der Bohrung nicht mehr gewechselt werden müssen, wird der Bohrprozess vereinfacht.

Ferner haben die Führungsbereiche aller Dentalbohrer des Sets von Dentalbohrern zusätzlich die gleiche Höhe. Die Höhe des Führungsbereichs hat einen entscheidenden Einfluss auf die

Bohrtiefe, die mit dem entsprechenden Dentalbohrer maximal erreicht werden kann. Aus diesem Grund ist es vorteilhaft, wenn alle Dentalbohrer eines Sets einen Führungsbereich mit identischer Höhe aufweisen, so dass diesbezüglich keine Verwechslungsgefahr besteht.

Vorzugsweise weist der Führungsbereich der Dentalbohrer einen Durchmesser von 2.6 bis 3.0 mm, insbesondere von 2.8 mm auf. Die Länge des Führungsbereichs der Dentalbohrer beträgt vorzugsweise etwa 2 bis 10 mm, insbesondere etwa 4 bis 6 mm. Damit ist der Führungsbereich optimal auf handelsübliche Bohrerführungen abgestimmt.

In einer bevorzugten Ausführungsform weist der Schaftteil der Dentalbohrer zusätzlich einen Bohrstopp auf. Alternativ ist es auch möglich, dass der Schaftteil zusätzlich eine Nute oder einen Wulst zur Befestigung eines Bohrstopps aufweist. Die Integration eines Bohrstopps oder einer Nute oder eines Wulstes zur Befestigung eines separaten Bohrstopps garantiert, dass beim Bohren eine bestimmte maximale Bohrtiefe nicht überschritten werden kann. Ein solcher Bohrstopp steht radial über den Schaft des Dentalbohrers hinaus und liegt auf der Bohrhülse oder der Reduktionshülse auf, sobald die gewünschte maximale Bohrtiefe erreicht wird. Ein weiteres Absenken des Dentalbohrers in das Bohrloch ist dann nicht möglich.

Erfindungsgemäss weist der Schaftteil der Dentalbohrer zusätzlich einen Einführbereich zum seitlichen Einführen der Dentalbohrer in eine Bohrerführung auf. Dieser Einführbereich ist schmaler gestaltet als der Führungsbereich. Je nach Breite des Schneideteils der Dentalbohrer ist der Einführbereich ausserdem auch schmaler als der Schneideteil der Dentalbohrer ausgestaltet. Damit ein seitliches Einführen der Dentalbohrer in eine Bohrerführung möglich ist, muss diese Bohrerführung einen seitlichen Schlitz aufweisen, der mindestens so breit ist wie der Einführbereich der Dentalbohrer. Durch die Ausgestaltung eines Einführbereichs an den Dentalbohrern ist es möglich, auch solche mit einem besonders dicken Schneideteil und insbesondere Profilbohrer in eine Bohrerführung einzuführen. Dadurch kann auch im Falle von Dentalbohrern, deren Schneideteil einen grösseren Durchmesser ausweist als der Innendurchmesser der Bohrerführung eine optimale Einhaltung der gewünschten Implantationsachse gewährleistet werden.

Vorzugsweise hat der Einführbereich einen Durchmesser von 1.2 bis 2.2 mm, insbesondere von 1.7 mm. Ein Einführbereich mit diesem Durchmesser ist genügend "dick" und damit genügend stabil, um ein Abbrechen des Bohrers unter den üblichen Belastungen des Bohrprozesses zu verhindern. Auf der anderen Seite sollte der Einführbereich aber deutlich schmaler als der Führungsbereich sein, so dass auch im Falle einer Bohrerführung mit einem seitlichen Schlitz, der natürlich an den Durchmesser des Einführbereichs angepasst sein muss, eine präzise Führung des Bohrers gewährleistet ist. Bohrer, deren Führungsbereich einen Durchmesser von etwa 2.8 mm und deren Einführbereich einen Durchmesser von etwa 1.7 mm aufweisen, erfüllen diese Anforderungen optimal.

In einer bevorzugten Ausführungsform umfasst das Set von Dentalbohrern mindestens einen Profilbohrer. In einer weiteren bevorzugten Ausführungsform umfasst das Set von Dentalbohrern mindestens einen Gewindebohrer. Die oben dargelegten Vorteile des Sets treten ganz besonders bei Profilbohrern und Gewindebohrern auf, die einen relativ breiten Schneideteil aufweisen.

In einer weiteren bevorzugten Ausführungsform umfasst das Set mindestens einen Rosenbohrer, einen Pilotbohrer, einen Profilbohrer, einen Spiralbohrer und einen Gewindebohrer. Ein solches Set ermöglicht es dem Dentalchirurgen, die gesamte Präparation des Implantationsbettes mit einem Set durchzuführen. Ausserdem kann für alle Bohrertypen die gleiche Bohrerführung verwendet werden.

In einer bevorzugten Ausführungsform weisen nicht alle Schneideteile der Dentalbohrer den gleichen Durchmesser auf. Ein solches Set mit Dentalbohrern unterschiedlichen Durchmessers erlaubt es, die schrittweise Erweiterung des Bohrlochs mit einem einzelnen Set durchzuführen. Ausserdem kann für alle Dentalbohrer des Sets die gleiche Bohrerführung verwendet werden. Vorzugsweise sind die verschiedenen Dentalbohrer des Sets je nach dem Durchmesser ihres Schneideteils markiert, beispielsweise durch eine Farbmarkierung. Ein solche (Farb-) Markierung kann zum Beispiel am Schaftteil der Dentalbohrer angebracht sein. Vorzugsweise umfasst das Set Dentalbohrer, deren Schneideteile einen Durchmesser von 3.3 mm, von 4.1 mm oder von 4.8 mm aufweisen.

Die vorliegende Erfindung betrifft ein Dentalimplantologieset, welches ein Set von Dentalbohrern sowie eine Bohrerführung umfasst. Mit dem Begriff "Bohrerführung" sind Bohrhülsen, Reduktionshülse, Bohrlöffel und ähnliche Hilfsmittel zur Führung des Dentalbohrers gemeint. Der Durchmesser des Führungsbereichs der Dentalbohrer entspricht dabei dem Innendurchmesser der Bohrerführung. Die Bohrerführung ist also auf die im Dentalimplantologieset ebenfalls enthaltenen Dentalbohrer abgestimmt.

In einer bevorzugten Ausführungsform weist das erfindungsgemässe Dentalimplantologieset zusätzlich einen Bohrstopp auf. Durch die Kombination des Sets von Dentalbohrern mit einer Bohrerführung und einem Bohrstopp kann die Präparation des Implantatbettes optimal gestaltet werden. Dabei erlaubt die Bohrerführung die Kontrolle der optimalen Implantationsachse, wahrend der Bohrstopp ein zu tiefes Eindringen in den Kieferknochen verhindert

Es zeigt:
- Fig. 1: ein Set von Dentalbohrern;
- Fig. 2: eine schematische Darstellung eines Dentalbohrers aus einem Set mit einer Bohrerführung;
- Fig. 3: eine schematische Darstellung des Bohrprozesses mit einem Dentalbohrers aus einem Set in Kombination mit einer Bohrschablone.

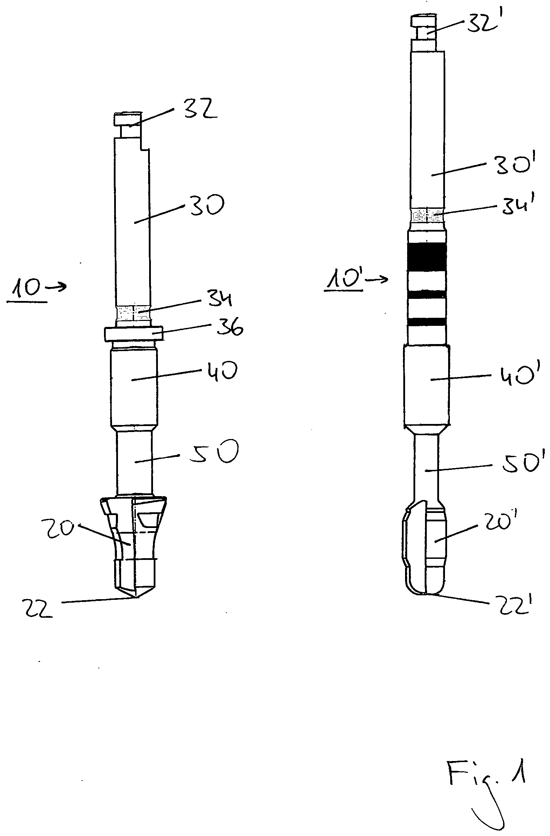

Figur 1 zeigt ein Set von zwei Dentalbohrern 10, 10', bestehend aus einem Profilbohrer 10 und einem Gewindebohrer 10'. Die beiden Dentalbohrer 10, 10' weisen einen Schneideteil 20, 20' und einen Schaftteil 30, 30' auf. Am unteren Ende des Schneideteils 20, 20' ist ein stirnseitiges Bohrende 22, 22' ausgebildet. Am oberen Ende des Schaftteils 30, 30' befindet sich ein Aufnahmeendbereich 32, 32', welcher dazu bestimmt ist, in einer Bohrerhaltevorrichtung aufgenommen zu werden. Der Schaftteil 30, 30' weist ausserdem einen im Wesentlichen kreiszylinderförmigen Führungsbereich 40, 40' zur Führung des Dentalbohrers 10, 10' während des Bohrprozesses. Die Führungsbereiche 40, 40' der beiden Dentalbohrer 10, 10' haben den gleichen Durchmesser. Der Schaftteil 30, 30' weist zusätzlich einen Einführbereich 50, 50' auf, der ein seitliches Einführen der Dentalbohrer in eine Bohrerführung ermöglicht. Durch eine Farbmarkierung 34, 34' am Schaftteil 30, 30' der Dentalbohrer 10, 10' ist der Durchmesser des jeweiligen Schneideteils 20, 20' kodiert. Der Profilbohrer 10 weist ausserdem einen Bohrstopp 36 auf, der eine maximale Bohrtiefe definiert.

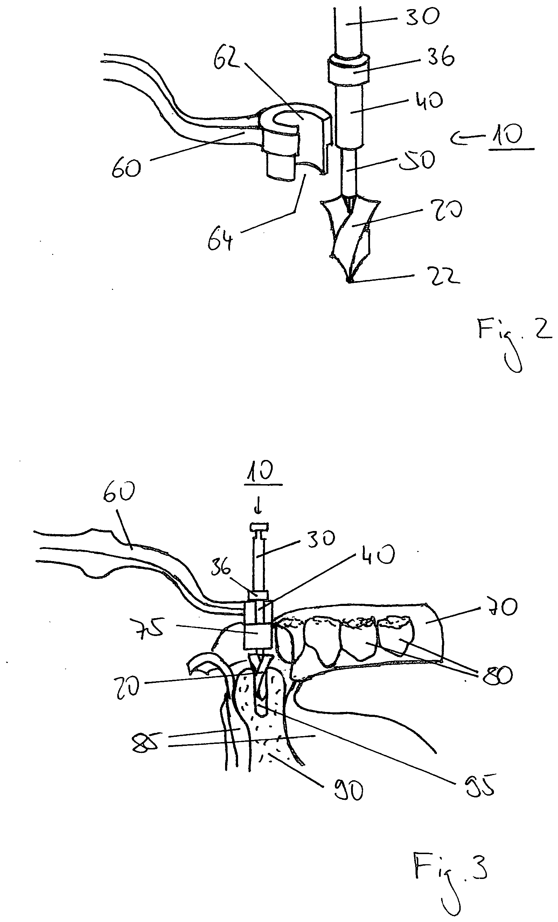

Figur 2 zeigt schematisch, wie ein Dentalbohrer 10 aus einem Set seitlich in eine Bohrerführung 60 eingeführt wird. Zu diesem Zweck wird der Einführbereich 50 des Dentalbohrers 10 durch einen seitlichen Schlitz 64 in der Führungshülse 62 der Bohrerführung 60 geführt. Durch anschliessendes Absenken .des Dentalbohrers 10 in der Bohrerführung 60 wird der Führungsbereich 40 des Dentalbohrers auf die Höhe der Führungshülse 62 gebracht. Wenn beim Bohren der Bohrstopp 36 des Dentalbohrers 10 auf den oberen Rand der Führungshülse 62 stösst, ist die gewünschte Bohrtiefe erreicht und der Dentalbohrer 10 kann nicht mehr weiter abgesenkt werden.

Figur 3 zeigt eine schematische Darstellung einer Bohrschablone 70, welche auf dem Gebiss 80 eines Patienten angebracht ist. Die Bohrschablone 70 weist an der vorbereiteten Implantationsstelle ein Bohrloch auf, in welchem eine Bohrhülse 75 befestigt ist. Rund um die Implantationsstelle ist das Weichgewebe 85 zurückgefaltet und der Kieferknochen 90 freigelegt, in welchem bereits eine Pilotbohrung 95 gemacht worden ist. Für die weitere Präparierung des Implantatbettes im Kieferknochen 90 wurde der Dentalbohrer 10 in die Bohrerführung 60 eingeführt und in die Bohrhülse 75 abgesenkt. Dadurch wird der Dentalbohrer 10 im Führungsbereich 40 während des Bohrprozesses geführt und ein Abrutschen oder Abknicken verhindert. Der Bohrstopp 36 stellt sicher, dass die gewünschte maximale Bohrtiefe nicht überschritten wird. In der Folge werden schrittweise weitere Dentalbohrer 10, 10' aus dem Set in die Bohrerführung 60 und die Bohrhülse 75 eingeführt und das Implantationsbett bis auf die gewünschte Grösse erweitert.

## Patentansprüche

1. Dentalimplantologieset, umfassend
ein Set von Dentalbohrern (10, 10') zur Präparation eines Implantatbettes, umfassend zwei oder mehr Dentalbohrer (10, 10'), und eine Bohrerführung (60, 60'),
wobei die Dentalbohrer (10, 10') einen Schneideteil (20, 20') mit einem stirnseitigen Bohrende (22, 22') und einen Schaftteil (30, 30') mit einem Aufnahmeendbereich (32, 32'), welcher dazu bestimmt ist, in einer Bohrerhaltevorrichtung aufgenommen zu werden, umfassen, wobei der Schaftteil (30, 30') einen im Wesentlichen kreiszylinderförmigen Führungsbereich (40, 40') aufweist zur Führung des Dentalbohrers (10, 10') und wobei die Führungsbereiche (40, 40') der zwei oder mehr Dentalbohrer (10, 10') den gleichen Durchmesser und die gleiche Höhe haben, wobei der Schaftteil (30, 30') der Dentalbohrer (10, 10') zusätzlich einen Einführbereich (50, 50') zum seitlichen Einführen der Dentalbohrer (10, 10') in eine Bohrerführung (60, 60') aufweist, welcher Einführbereich (50, 50') schmaler gestaltet ist als der Führungsbereich (40, 40') und als der Schneideteil (20, 20') der Dentalbohrer (10, 10'), **dadurch gekennzeichnet, dass**
der Durchmesser des Führungsbereichs (40, 40') der Dentalbohrer (10, 10') dem Innendurchmesser der Bohrerführung (60, 60') entspricht,
dass die Bohrerführung (60, 60') einen seitlichen Schlitz aufweist, der mindestens so breit ist wie der Einführbereich (50, 50') der Dentalbohrer (10, 10'), dass der Schneideteil (20, 20') der Dentalbohrer (10, 10') einen grösseren Durchmesser aufweist als der Innendurchmesser der Bohrerführung (60, 60'), und dass das Set mindestens einen Profilbohrer (10) und/oder einen Gewindebohrer (10') umfasst.

2. Dentalimplantologieset nach einem der vorangehenden Ansprüche, **dadurch gekennzeichnet, dass** der Führungsbereich (40, 40') der Dentalbohrer (10, 10') einen Durchmesser von 2.6 bis 3.0 mm aufweist, insbesondere von 2.8 mm.

3. Dentalimplantologieset nach einem der vorangehenden Ansprüche, **dadurch gekennzeichnet, dass** der Führungsbereich (40, 40') der Dentalbohrer (10, 10') eine Länge von etwa 4 bis 6 mm aufweist.

4. Dentalimplantologieset nach einem der vorangehenden Ansprüche, **dadurch gekennzeichnet, dass** der Schaftteil (30, 30') der Dentalbohrer (10, 10') zusätzlich einen Bohrstopp (34, 34') aufweist.

5. Dentalimplantologieset nach einem der Ansprüche 1 bis 3, **dadurch gekennzeichnet, dass** der Schaftteil (30, 30') der Dentalbohrer (10, 10') zusätzlich eine Nut oder einen Wulst zur Befestigung eines Bohrstopps aufweist.

6. Dentalimplantologieset nach einem der vorangehenden Ansprüche, **dadurch gekennzeichnet, dass** der Einführbereich (50, 50') einen Durchmesser von 1.2 bis 2.2 mm aufweist, insbesondere von 1.7 mm.

7. Dentalimplantologieset nach einem der vorangehenden Ansprüche, **dadurch gekennzeichnet, dass** das Set mindestens einen Rosenbohrer, einen Pilotbohrer, einen Profilbohrer (10), einen Spiralbohrer und einen Gewindebohrer (10') umfasst.

8. Dentalimplantologieset nach einem der vorangehenden Ansprüche, **dadurch gekennzeichnet, dass** nicht alle Schneideteile (20, 20') der Dentalbohrer (10, 10') den gleichen Durchmesser aufweisen.

9. Dentalimplantologieset nach Anspruch 1, **dadurch gekennzeichnet, dass** es zusätzlich einen Bohrstopp umfasst.

## Claims

1. Dental implantology set, comprising a set of dental drills (10, 10'), for the preparation of an implant bed, comprising two or more dental drills (10, 10'), and a drill guide, wherein the dental drills (10, 10') comprise a cutting portion (20, 20') having an end side-proximal drill end (22, 22') and a shank portion (30, 30') having a receptacle end region (32, 32') which is specified to be received in a drill holding device, wherein the shank portion (30, 30') has a substantially circular-cylindrical guide region (40, 40') for guiding the dental drill (10, 10'), and wherein the guide regions (40, 40') of the two or more dental drills (10, 10') are of the same diameter and of the same height,
wherein the shank portion (30, 30') of the dental drills (10, 10') additionally has an introduction region (50, 50') for laterally introducing the dental drills (10, 10') into a drill guide (60, 60'), said introduction region (50, 50') being designed so as to be narrower than the guide region (40, 40') and the cutting portion (20, 20') of the dental drills (10, 10'),
**characterized in that** the diameter of the guide region (40, 40') of the dental drills (10, 10') corresponds to the internal diameter of the drill guide (60, 60'),
that the drill guide (60, 60') comprises a lateral slot, said lateral slot being at least as wide as the introduction region (50, 50') of the dental drills (10, 10'), wherein the cutting portion (20, 20') of the dental drills (10, 10') has a larger diameter than the internal diameter of the drill guide (60, 60'),
and that the set includes at least one profile drill (10) and/or one thread tap drill (10').

2. Dental implantology set according to one of the preceding claims, **characterized in that** the guide region (40, 40') of the dental drills (10, 10') has a diameter of 2.6 to 3.0 mm, in particular of 2.8 mm.

3. Dental implantology set according to one of the preceding claims, **characterized in that** the guide region (40, 40') of the dental drills (10, 10') has a length of approximately 4 to 6 mm.

4. Dental implantology set according to one of the preceding claims, **characterized in that** the shank portion (30, 30') of the dental drills (10, 10') additionally has a drilling stop (34, 34').

5. Dental implantology set according to one of claims 1 to 3, **characterized in that** the shank portion (30, 30') of the dental drills (10, 10') additionally has a groove or a bead for fastening a drilling stop.

6. Dental implantology set according to one of the preceding claims, **characterized in that** the introduction region (50, 50') has a diameter of 1.2 to 2.2 mm, in particular of 1.7 mm.

7. Dental implantology set according to one of the preceding claims, **characterized in that** the set comprises at least one bud bur drill, one pilot drill, one profile drill (10), one spiral drill and one thread tap drill (10').

8. Dental implantology set according to one of the preceding claims, **characterized in that** not all cutting portions (20, 20') of the dental drills (10, 10') are of the same diameter.

9. Dental implantology set according to Claim 1, **characterized in that** said dental implantology set additionally comprises a drilling stop.

## Revendications

1. Ensemble d'implantologie dentaire, comprenant un ensemble de fraises dentaires (10, 10') destiné à la préparation d'un site d'implant, ledit ensemble comprenant deux fraises dentaires (10, 10') ou plus, et un guide-fraise (60, 60'), les fraises dentaires (10, 10') comprenant une partie de coupe (20, 20') pourvue d'une extrémité de fraisage frontale (22, 22') et une partie formant tige (30, 30') pourvue d'une zone d'extrémité de réception (32, 32') qui est destinée à être reçue dans un dispositif porte-fraise, la partie formant tige (30, 30') comportant une zone de guidage (40, 40') qui est sensiblement en forme de cylindre à base circulaire et qui est destinée à guider la fraise dentaire (10, 10') et les zones de guidage (40, 40') des deux fraises dentaires (10, 10') ou plus ayant le même diamètre et la même hauteur, la partie formant tige (30, 30') de la fraise dentaire (10, 10') comportant en outre une zone d'insertion (50, 50') destinée à insérer latéralement la fraise dentaire (10, 10') dans un guide de fraise (60, 60'), laquelle zone d'insertion (50, 50') étant plus étroite que la zone de guidage (40, 40') et que la partie de coupe (20, 20') des fraises dentaires (10, 10'),
**caractérisé en ce que** le diamètre de la zone de guidage (40, 40') des fraises dentaires (10, 10') correspond au diamètre intérieur du guide-fraise (60, 60'),
que le guide-fraise (60, 60') présente une fente latérale qui est au moins aussi large que la zone d'insertion (50, 50') des fraises dentaires (10, 10'),
que la partie de coupe (20, 20') présente un diamètre plus grand que le diamètre intérieur du guide-fraise(60, 60'),
et que l'ensemble comprend au moins une fraise profilée (10) et/ou une fraise filetée (10').

2. Ensemble d'implantologie dentaire selon l'une des revendications précédentes, **caractérisé en ce que** la zone de guidage (40, 40') des fraises dentaires (10, 10') a un diamètre de 2,6 à 3,0 mm, notamment de 2,8 mm.

3. Ensemble d'implantologie dentaire selon l'une des revendications précédentes, **caractérisé en ce que** la zone de guidage (40, 40') des fraises dentaires (10, 10') a une longueur d'environ 4 à 6 mm.

4. Ensemble d'implantologie dentaire selon l'une des revendications précédentes, **caractérisé en ce que** la partie formant tige (30, 30') des fraises dentaires (10, 10') comporte en outre une butée de fraisage (34, 34').

5. Ensemble d'implantologie dentaire selon l'une des revendications 1 à 3, **caractérisé en ce que** la partie formant tige (30, 30') des fraises dentaires (10, 10') comporte en outre une rainure ou un bourrelet destiné à fixer une butée de fraisage.

6. Ensemble d'implantologie dentaire selon l'une des revendications précédentes, **caractérisé en ce que** la zone d'insertion (50, 50') a un diamètre de 1,2 à 2,2 mm, notamment de 1,7 mm.

7. Ensemble d'implantologie dentaire selon l'une des revendications précédentes, **caractérisé en ce que** l'ensemble comprend au moins une fraise boule, une fraise pilote, une fraise profilée (10), une fraise en spirale et une fraise filetée (10').

8. Ensemble d'implantologie dentaire selon l'une des revendications précédentes, **caractérisé en ce que** toutes les parties de coupe (20, 20') des fraises dentaires (10, 10') n'ont pas le même diamètre.

9. Ensemble d'implantologie dentaire selon la revendication 1, **caractérisé en ce qu'**il comprend en outre une butée de fraisage.
